# EUROPÄISCHE PATENTSCHRIFT

(11) **EP 1 578 255 B1**
(45) Veröffentlichungstag und Bekanntmachung des Hinweises auf die Patenterteilung: **06.09.2006**
(21) Anmeldenummer: 03788857.5
(22) Anmeldetag: 05.12.2003
(51) Int. Cl.: A61B 1/00

(54) **HYGIENESCHUTZ FÜR ENDOSKOPE**
HYGIENE PROTECTION FOR ENDOSCOPES
ELEMENT DE PROTECTION HYGIENIQUE POUR ENDOSCOPE

(30) Priorität: 03.01.2003 DE 20300037 U; 13.05.2003 DE 10321313; 31.10.2003 DE 20316892 U
(43) Veröffentlichungstag der Anmeldung: 28.09.2005
(73) Patentinhaber: Kress, Jürgen, 84051 Essenbach (DE)
(72) Erfinder: Kress, Jürgen, 84051 Essenbach (DE)
(74) Vertreter: Pöhner, Wilfried Anton, Dr.
(86) Internationale Anmeldenummer: PCT/DE2003/004017
(87) Internationale Veröffentlichungsnummer: WO 2004/060149

(56) Entgegenhaltungen:
- EP-A- 0 310 515
- WO-A-94/05200
- DE-A- 19 918 488
- US-A- 4 646 722
- US-A- 4 907 395
- US-A- 5 217 001
- US-A- 5 702 348

## Beschreibung

Die Erfindung betrifft Endoskop mit Hygieneschutz, bestehend aus einer Hülle, die am distalen Ende geschlossen sowie zumindest stirnseitig für optische Informationen durchlässig ist, und die in Achsrichtung des Endoskops kondomartig aufrollbar ist, und aus einem oder mehreren parallel zum Endoskop verlaufenden Arbeitskanälen, die am distalen Ende der Hülle offen enden, wobei die Arbeitskanäle nur mit dem distalen Ende der Hülle verbunden sind und die Arbeitskanäle zwischen der Außenseite des Endoskops und der Innenseite der Hülle positioniert sind. Die Erfindung betrifft außerdem ein Verfahren zum Anlegen eines elastischen Endoskopschutzes, der zur einmaligen Nutzung vorgesehen ist.

Die Endoskopie ist ein diagnostisches. Verfahren zur Untersuchung von Körperhöhlen und - kanälen sowie Hohlorganen durch unmittelbare Betrachtung mit Hilfe eines Endoskops. Endoskope neuerer Bauart bestehen aus einem biegsamen Rohr, in dessen Innerem Glasfaserbündel verlaufen- Die optische Information aus dem Körperinneren wird durch die Glasfasern übertragen. Andere Endoskope verfügen über einen als miniaturisierte Fernsehkamera dienenden CCD Bildwandler Chip, der eine Bildschirmwiedergabe ermöglicht. Mit Hilfe der Endoskopie können durch einführbare Zangen und Schlingen, Spül- und Absaugvorrichtungen zur Biopsie Gewebsteile entnommen und kleinere operative Eingriffe vorgenommen werden.

Da Endoskope sehr teuere Geräte sind, ist zu ihrer Amortisierung ein möglichst häufiger Einsatz erforderlich. Zur Vermeidung von Kontamitationen müssen ungeschützte Endoskope nach jeder Nutzung zerlegt und gründlich gereinigt werden. Die Reinigungsprozedur schließt die intensive mechanische Säuberung z.B. durch Bürsten sowie den Einsatz eines Ultraschallbades ein Die im Inneren des Endoskops verlaufenden Kanäle werden nach Einlage in eine Desinfektionslösung mit Hilfe einer Pumpe gespült. Die gesamte Prozedur erfordert einen hohen Aufwand an Zeit und Personal. Außerdem ist das Endoskop für den Zeitraum der Reinigung nicht einsatzfähig, so dass unter vermeidbarer Kapitalbindung zusätzliche Endoskope angeschafft werden müssen, um die Patientenversorgung zu sichern.

Zur Umgehung dieser Schwierigkeiten wurden bereits verschiedene Endoskophüllen zur einmaligen Nutzung (US 4741326), (US 005217001 A), (US 5 201 908), (DE 199 18 488) vorgeschlagen. Trotz des nachweislich großen Bedarfs für Endoskopieschutzhüllen hat sich interessanterweise keine dieser Hüllen bislang am Markt durchgesetzt.

Das Dokument (US 005217001 A) offenbart verschiedene Ausführungsformen einer Endoskopschutzhülle: In einer der Ausführungsformen besteht die Hülle aus einem starren Material, das bei der Anbringung der Hülle in seiner gesamten Länge über die distale Spitze des Endoskops geschoben werden muss. Zur Erleichterung dieses Vorgangs ist der Innendurchmesser der Hülle größer bemessen als der Außendurchmesser des Endoskops. Nachdem das distale Ende des Endoskops am distalen Ende der Hülle anliegt wird der nicht auf der Außenseite des Endoskops anliegende Teil der Hülle umgeschlagen, wobei zur Fixierung der unbeschlagenen Hülle in Längsrichtung und auf dem Nut- und - Federprinzip basierende reissverschlussartige Verschlüsse vorgesehen sind. Nachteilig an dieser Ausführungsform ist neben der umständlichen Anbringung dieser Hülle ihre Starrheit sowie die, die zur Anbringung der in Längsrichtung verlaufenden Verschlüsse höheren Herstellungskosten. In einer Weiterbildung dieses Endoskopschutzes weist die Hülle keinen reißverschlussartigen Verschluss, sondern eine auf der Innenseite der Hülle anliegende luftdichte ballonartige Hülle auf. Diese ballonartige Hülle kann mit Luft beaufschlagt werden, sodass der Endoskopschutz fest am Schaft des Endoskops anliegt. Der wesentliche Nachteil dieser Ausführungsform besteht in der Volumenzunahme des Endoskops, denn im Interesse des Patienten ist jede Volumenzunahme des Endoskops zu vermeiden, da die Einführung eines Endoskops mit großem Durchmesser sehr schmerzhaft sein kann. Die eher starre Hülle der beiden genannten Ausführungsformen weist in der Wand der Hülle parallel zum Endoskopschaft verlaufende Röhren auf, die als Arbeitskanäle dienen.

In einer alternativen Ausführungsform zeigt (US 005217001 A) einen kondomartig abrollbaren Endoskopschutz, der am distalen Ende, genauso wie die beiden aus diesem Dokument erstgenannten Ausführungsformen, ein transparentes Fenster aufweist. Zusätzliche Arbeitskanäle sind bei dem kondomartig abrollbaren Endokopschutz nicht vorgesehen. Sein Einsatz beschränkt sich daher nachteiligerweise auf die optische Kontrolle und schließt die Entnahme von Biopsien aus.

Die Druckschrift 1 (US-A-4 646 722) beschreibt einen Endoskopschutz, der aus einem elastischen Material besteht und ausgehend vom distalen Ende kondomartig auf der Außenseite des Endoskops abrollbar ist. Die Hülle ist am distalen Ende für optische Informationen durchlässig und weist in diesem Bereich offen endende Arbeitskanäle auf. Nach Fixierung des Endoskopschutzes am Endoskop liegen die Arbeitskanäle zwischen der Außenseite des Endoskops und der Innenseite der Hülle und werden durch diese fixiert.

Das Dokument 2 (WO 94 05200 A) offenbart ein Endoskop, das am distalen Ende die lösbare Befestigung einer eigenständigen Kanal-Einheit erlaubt. Die Kanal-Einheit besteht aus einem Endstück, das beispielsweise mit Hilfe eines Schwalbenschwanzprofils an einer am distalen Ende des Endoskops dazu komplementär zum Schwalbenschwanzprofil geformten Vertiefung befestigt wird. Die Öffnungen der Arbeits- und Biopsiekanäle befinden sich am distalen Ende der Kanal-Einheit, in die sie eingelassen sind. Nach Verlassen der Kanal-Einheit verlaufen die Arbeits- und Biopsykanäle parallel zum Endoskopschlauch. Eine zusätzliche Befestigung, insbesondere durch einen abrollbaren, das Endoskop und die Kanal-Einheit umgebenden kondomartigen Schutz aus Gummi, ist nicht vorgesehen.

Druckschrift D3 (DE 199 18 488 A1) beschreibt einen wegwerfbaren Endoskopschutz, der als flüssigkeitsdichte Hülle das Endoskop umschließt und am distalen Ende ein für optische Informationen transparentes Fenster aufweist, wobei der optische Kontakt zwischen dem Fenster der Hülle und dem optischen Kanal des Endoskops durch die Einbringung einer transparenten Flüssigkeit verbessert wird,

Die Aufgabe der Erfindung besteht vor diesem Hintergrund in der Konstruktion eines mit geringem Aufwand schnell fixierbaren Hygieneschutzes mit Arbeitskanälen zum einmaligen oder mehrmaligen Gebrauch für medizinische Endoskope, wobei die Erweiterung des Endoskopdurchmessers weitgehend oder ganz vermieden werden soll.

Zur Lösung der Aufgabe wird ein Hygieneschutz vorgeschlagen, der dadurch gekennzeichnet ist, dass neben den Arbeitskanälen ein oder mehrere Vakuumkanäle mit einer oder mehreren Öffnungen, die auf der dem Endoskop zugewandten Innenseite der hülle enden, vorgesehen sind.

Die Arbeitskanäle sind beim erfindungsgemäßen Endoskopschutz mit dem distalen Ende der Hülle verbunden; im nicht-distalen Bereich der Hülle sind die Arbeits- und Vakuumkanäle sozusagen freiliegend zwischen dem Endoskopschaft und der Innenseite der Hülle fixiert. Im allgemeinsten Fall sind die Arbeitskanäle innenseitig nur mit der Stirnseite der distalen Kappe luft- und wasserdicht verbunden. Das Innere der Arbeitskanäle ist somit nur von der Außenseite des Endoskopschutzes zugänglich.

Neben den Arbeitskanälen ist beim erfindungsgemäßen Endoskopschutz mindestens ein Vakuumkanal vorgesehen. Dieser Vakuumkanal endet innerhalb der Hülle des Endoskopschutzes offen und kann zusätzliche seitliche Öffnungen aufweisen. Diese seitlichen Öffnungen enden auf der Innenseite der Hülle. Bei Anlegen eines Vakuums an diesen Kanal wird die zwischen Hülle und Endoskopschaft befindliche Luft abgesaugt mit der Folge, dass die Hülle fest an das Endoskop gezogen wird. Das Vakuum bleibt während der Untersuchung bestehen. So wird vorteilhafterweise schnell eine feste Verbindung zwischen Hülle und Endoskop hergestellt, nachdem das Endoskop bequem in die im Innendurchmesser, vorzugsweise im proximalen Teil, etwas größer bemessene Hülle eingeführt wurde.

Beim Anlegen des Hygieneschutzes fixiert eine Hand die freibeweglichen Arbeits- und Vakuumkanäle am Endoskopschaft, während die andere Hand die kondomartig aufgerollte oder balkartig zusammengefalltete Hülle oberhalb der Kanäle abrollt.

Der Kerngedanke der Erfindung besteht in der Kombination der kondomartigen Schutzhülle mit eigenständigen Arbeitskanälen, die außerhalb des Endoskops verlaufen, wobei die Schutzhülle und die Arbeitskanäle im distalen Bereich der Hülle luft-, keim-, und wasserdicht miteinander verbunden sind. Zur Anbringung der erfindungsgemäßen Schutzhülle wird das distale Ende der Hülle auf das Endoskop gesteckt, sodass das die für optische Informationen durchlässige Stirnseite korrekt, d. h. parallel zum distalen Ende des Endoskops positioniert ist. Der optische Kontakt zwischen Endoskop und der transparenten Stirnseite der Hülle wird durch eine Flüssigkeit wie z. B. Mikroskopieröl, die idealerweise den gleichen Brechungsindex wie die Linse des Endoskops aufweist, hergestellt. Bei Anbringung des Endoskopschutzes werden die Kanäle mit einer Hand am Endoskop fixiert, während die andere Hand die kondomartig aufgerollte oder ballartig zusammengefalltete Hülle über die Außenseite des Kondoms schiebt, so dass die Hülle neben dem Endoskop auch die Arbeitskanäle umschließt.

Es liegt im Rahmen der Erfindung, dass der Hygieneschutz für medizinische Endoskope eingesetzt wird, deren Durchmesser variabel sein kann. Neben Endoskopen zur Untersuchung der Speiseröhre und aller Verdauungsorgane sind ausdrücklich Endoskope eingeschlossen, die in der Hals-Nasen-Ohren Medizin eingesetzt werden.

Die Hülle des erfindungsgemäßen Endoskopschutzes soll aus einem flexiblen Material bestehen, das luft- und wasserdicht ist und außerdem für krankheitsbildende Keime undurchlässig ist das gleiche gilt für die Art der Verbindung der Arbeitskanäle mit der Hülle an deren distalem Ende. In der Regel erfüllen die, für medizinische Zwecke eingesetzten gummiartigen Materialien diese Erfordernisse. Derartige Materialien weisen zum Teil thermosensitive Eigenschaften auf, d.h. ihre Ausdehnung verringert sich bei Erwärmung. Zur vorteilhaften Nutzung dieser Eigenschaften im Zusammenhang mit dem erfindungsgemäßen Endoskop wäre denkbar, dass das Endoskop nach Anbringung der Schutzhülle mit oder ohne das Anliegen eines Unterdrucks am Vakuumschlauch eine kurze Erwärmung erfolgt. Bei vorliegen der genannten thermosensitiven Materialeigenschaften des Endoskopschutzes hätte ein derartiger Verfahrensschritt die vorteilhafte Wirkung, dass der Endoskopschutz faltenfrei am Schaft des Endoskopes anläge.

In einer Weiterbildung des erfindungsgemäßen Endoskopschutzes ist vorgesehen, dass die Hülle nicht kondomartig abrollbar, sondern balgartig zusammenfaltbar ist. Diese Weiterbildung ist insbesondere dann anwendbar, wenn die Oberfläche des Endoskops glatt und ohne überstehende Teile ist.

Es liegt im Rahmen der Erfindung, dass der Innendurchmesser der Hülle, zumindest im proximalen Teil der Hülle, geringfügig größer ist, als der Außendurchmesser des Endoskops. Dadurch ist es vorteilhafterweise leicht möglich, die Hülle am Endoskop anzulegen. Dies gilt insbesondere, wenn die Hülle balkartig zusammenfaltbar ist.

Natürlich deckt die Hülle als Kontaminationsschutz zumindest die in die Körperöffnungen des Patienten eingeführten Teile des Endoskops ab. Zur Vermeidung jeglicher Kontaminationen des Endoskops wird die Hülle in der Praxis jedoch so bemessen sein, dass sie auch Bereiche des Endoskops abdeckt, die nicht mit dem Patienten in Kontakt kommen.

In einer bevorzugten Ausführungsform ist das für optische Informationen durchlässige Stirnseite am distalen Ende der Hülle eine transparente Scheibe oder eine Linse, die der Vergrößerung der optischen Information dient. Der optische Kontakt zwischen der Scheibe oder der Linse und dem optischen Kanals des Endoskops wird durch eine Flüssigkeit hergestellt, so dass die Sichtschärfe nicht beeinträchtig wird. Natürlich ist denkbar, dass die Scheibe oder die Linse die gesamte Fläche der Stirnseite am distalen Endabschnitts bildet. Prinzipiell ist auch denkbar, dass der distale Endabschnitt der Hülle als optisch transparente Kappe ausgebildet ist und stirnseitig und/oder seitlich eine oder mehrere Linsen fixiert. Die Wandstärke des kappenartigen Endabschnitts ist dabei größer, als die Wandstärke der Hülle im nicht-distalen Bereich. Der nicht-distale Bereich reicht vom proximalen Ende der Kappe bis zum proximalen Ende der Hülle.

Es entspricht der Lehre der Erfindung, dass die am proximalen Ende offene Hülle am Endoskopschaft luftdicht fixierbar ist, sodass beim Anlegen des Vakuums keine Luft von außen nachströmen kann. Eine entsprechende Abdichtung zwischen dem Endoskopschaft und der Hülle kann erreicht werden, indem die dazwischen liegenden Kanäle in eine gummiartige Manschette eingebettet werden.

In einer alternativen Ausführungsform der Hülle, ist diese im Bereich des proximalen Endes konisch aufgeweitet. Dies erleichtert die Anbringung der Hülle und erlaubt die zusätzliche Abdeckung von Bestandteilen des Endoskops, die über den Endoskopschaft hinausragen. Durch das faltenfreie Umschlagen der Hülle, wird diese vorzugsweise unter Spannung an den Schaft angelegt und mittels eines chemisch inerten und vor allem nicht toxischen Adhäsionskleber fixiert.

Zur leichteren Entfernung des Hygieneschutzes vorgesehen, dass auf der Innenseite der Hülle über deren gesamte Länge oder einen Teil ihrer Länge ein Reißfaden verläuft. Dieser Reißfaden ist an seinem distalen Ende mit der Hülle verbunden. Durch Zug in eine zur Endoskopachse senkrecht zeigende Richtung übt der wie eine Schnittkante wirkende Reißfaden eine Kraft auf die Hülle aus, welche dieselbe durchtrennt. Derartige Reißfäden sind dem Prinzip nach von Verpackungsmitteln bekannt.

Es liegt im Rahmen der Erfindung, dass mindestens einer der Arbeitskanäle zur Einleitung von Flüssigkeiten oder Luft vorgesehen ist, die so genutzten Kanäle müssen nicht zwingend starrwandig sein. Die Starrwandigkeit ist dagegen ein Erfordernis, das an einen als Saugleitung genutzten Arbeitskanal gestellt werden kann. Im Rahmen der Erfindung ist mindestens einer der Arbeitskanäle als Saugleitung zur Entnahme von Körperflüssigkeiten vorgesehen. Weiterhin ist mindestens einer der Arbeitskanäle zur Führung von Geräten d. h. insbesondere von Geräten, die der Entnahme von Gewebeproben (Biopsien) dienen vorgesehen.

Die erfindungswesentlichen Vakuumkanäle können sich über die gesamte Länge der Endoskophülle oder nur über einen Teil der Hülle erstrecken. Es ist denkbar, dass die elastische Hülle ausgehend vom distalen Ende bis z. B. zum 1. Drittel sehr eng bemessen ist, sodass sie bereits ohne das Anlegen eines Vakuums eng am Endoskopschaft anliegt. In diesem Fall wird sich der Vakuumkanal ausgehend vom proximalen Ende der Hülle nur über zwei Drittel der Hüllenlänge erstrecken. Dieser Bereich wird nach Anlegen des Vakuums vollends am Endoskopschaft anliegen.

Es liegt im Rahmen der Erfindung, dass während der Nutzung des Endoskops an dem oder an den Vakuumkanälen ein Unterdruck anliegt. Aufgrund der Abdichtung der Hülle am proximalen Ende ist es jedoch nicht zwingend erforderlich, dass der Unterdruck während der Endoskopnutzung beständig anliegen muss.

In einer Weiterbildung des erfindungsgemäßen Endoskops weist dieses auf der Außenseite des Endoskopschaftes Vertiefungen auf, die in axialer Richtung verlaufen. Die Form und Tiefe dieser Vertiefungen entspricht dem Durchmesser und Profil der Arbeits- und Vakuumkanäle, die nach Einlage in die Vertiefungen nicht mehr über die Oberfläche des Endoskopschaftes hinausragen. Dies erleichtert das Fixieren der Kanäle während des Anlegens der Hülle und ist zudem patientenfreundlich. In einer Weiterbildung dieser Ausführungsform, ist vorgesehen, dass die Breite der Vertiefungen im Endoskopschaft geringfügig schmäler ist, als die Breite der Kanäle. Da die Kanäle vorzugsweise aus elastischem Material, wie z. B. aus dehnbarem Plastik bestehen, lassen sie sich unter leichtem Druck in die Vertiefung am Endoskopschaft pressen und verbleiben dort, sodass sie vorteilhafterweise während des Abrollens bzw. des Überstreifens der Hülle nicht länger von Hand fixiert werden müssen.

In einer Weiterbildung der Erfindung sind die Arbeits- und Vakuumkanäle mit dem distalen Ende der Hülle lösbar verbunden. In diesem Fall sind adapterartige Steckverbindungen im distalen Endbereich der Hülle eingelassen, wobei die Adapter eine luft- und wasserdichte Steckverbindung ermöglichen. Luftdicht ist natürlich auch die Verbindung zwischen den Arbeits- und Vakuumkanälen am proximalen Ende. Es versteht sich von selbst, dass die adapterartigen Verbindungselemente der Arbeits- und Vakuumkanäle am proximalen Ende mit den in der Endoskopie gängigen und dem Fachmann bekannten Ventitanschlüssen und Endoskopsteuereinheiten kompartibel sind. Insbesondere sollen die Arbeits- und Vakuumkanäle auch an die aus dem Stand der Technik bekannten Endoskopsteuereinheiten, die nur zur einmaligen Nutzung vorgesehen sind, anschließbar sein. Derartige dem Fachmann bekannten Steuereinheiten sind vorzugsweise aus Plastik gestaltet.

Die vorliegende Erfindung umfasst auch ein Verfahren zur Anbringung eines wegwerfbaren Hygieneschutzes an medizinischen Endoskopen, wobei das Verfahren durch folgende Verfahrensschritte gekennzeichnet ist:
- das Fenster am distalen Ende der Hülle (4) innenseitig mit einem Stoff beaufschlagt wird, der den optischen Kontakt zwischen Fenster und dem optischen Kanal des Endoskops (2) herstellt,
- das distale Ende des Endoskops (2) in die am proximalen Ende offene und am distalen Ende geschlossene Hülle (4) eingeführt wird,
- die am distalen Ende der Hülle befestigten Arbeitskanäle (6a-6c) in den auf der Aussenseite des Endoskops (2) vorgesehenen Vertiefungen positioniert werden,
- die kondomartig aufgerollte oder balgartig zusammengefaltete Hülle (4) unter Einschluß des Endoskops (2) und der Arbeitskanäle (6a bis 6c) abgerollt oder entfaltet wird,
- am Vakuumschlauch ein Unterdruck angelegt wird.

Falls der Schaft des Endoskops auf seiner Außenseite keine Vertiefungen aufweist, entfällt der Verfahrensschritt Nummer 3.

Im folgendem sollen weitere Einzelheiten und Merkmale des erfindungsgemäßen Endoskopschutzes anhand von Beispielen näher erläutert werden. Das abgebildete Beispiel soll die Erfindung jedoch nicht einschränken, sondern diese nur erläutern. Es zeigen in schematischer Darstellung:
- **Figur 1**: Endoskopschutz vor Befestigung am Endoskop
- **Figur 2**: Endoskopschutz nach Anbringung der distalen Kappe am Endoskop
- **Figur 3**: Endoskop mit teilweise abgerolltem Endoskopschutz.

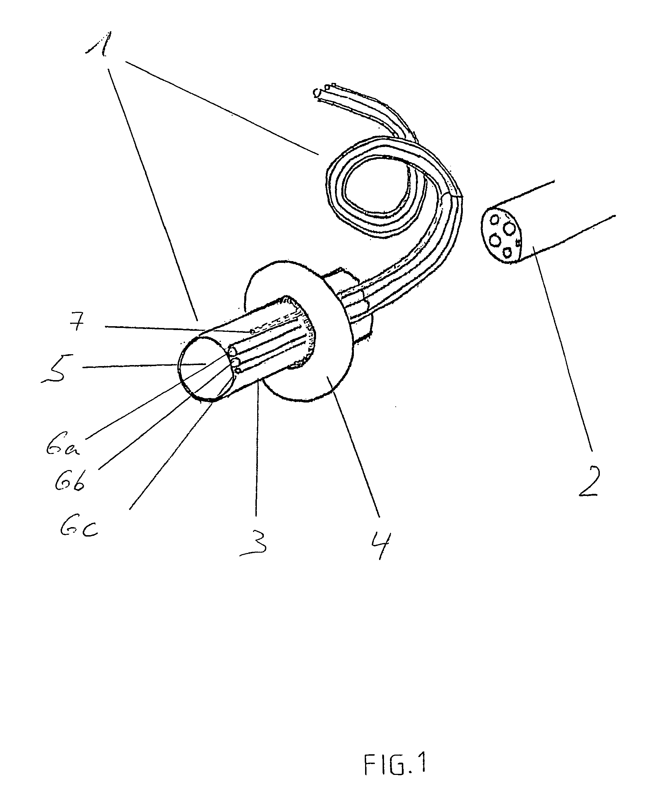

Figur 1 zeigt den aus dem distalen Endabschnitt 3 und aus der aufgerollten Hülle 4 sowie aus den Kanälen 7, 6a-6c bestehenden Endoskopschutz 1. Im gezeigten Beispiel ist das distale Ende 3 des Endoskopschutzes 1 kappenartig gestaltet. An der Stirnseite 5 befindet sich ein transparentes Fenster oder eine Linse, die ohne Einschränkung der Sichtschärfe die Übertragung der optischen Information zur Linse des Endoskops 2 zulässt. Die Arbeitskanäle 6a-6c sind mit dem distalen Ende 3 der Schutzhülle so verbunden, dass die Öffnungen der Arbeitskanäle 6a-6c auf der Außenseite der Schutzhülle 1 liegen. Alternativ wäre denkbar, dass die Arbeitskanäle 6a-6c auf der Innenseite des kappenartigen distalen Endabschnitts 3 anliegen und den Endabschnitt 3 an der Stirnseite 5 durchdrängen, wobei der stirnseitige Bestandteil des Endabschnitts 3 die Arbeitskanäle 6a-6c-luft- und wasserdicht umschließt.

Auf der Innenseite des distalen Endabschnitts verläuft der offen endende Vakuumkanal 7. Zusätzlich zum offenen Ende kann der Vakuumkanal 7 Öffnungen aufweisen, die auf der Innenseite der Endoskophülle 3 und 4 enden.

In dem über den distalen Endabschnitt 3 hinausgehenden Teil, sind die Arbeitskanäle 6a-6c sowie der Vakuumkanal 7 freiliegend.

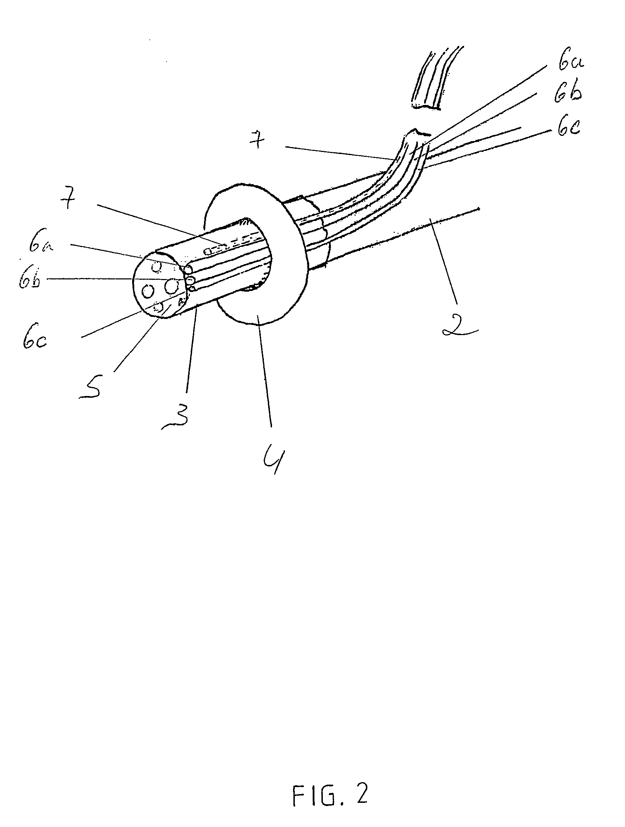

Figur 2 zeigt die in Figur 1 beschriebene Schutzhülle nachdem der distale Endabschnitt 3 der Schutzhülle auf das distale Ende des Endoskops 2 aufgeschoben wurde. Im gezeigten Stadium sind die Arbeitskanäle 6a-6c sowie der Vakuumkanal 7 indem über dem distalen Endabschnitt hinausgehenden Teil noch freiliegend.

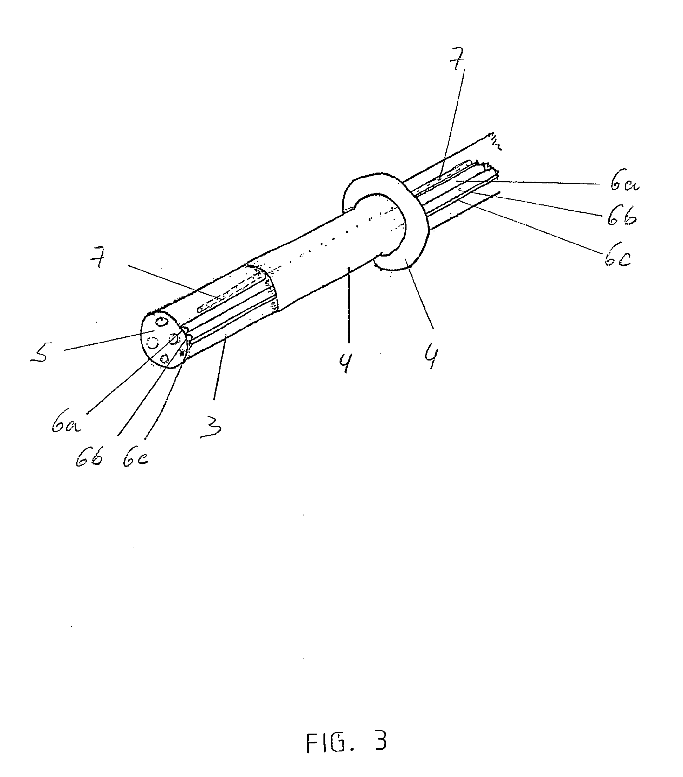

In Figur 3 ist gezeigt, wie die kondomartig aufgerollte Hülle 4 nach abrollen die am Endoskop 2 anliegenden Arbeitskanäle 6a-6c sowie den Vakuumkanal 7 nach dem Abrollen in Richtung des proximalen Endes des Endoskops 2 überdeckt. Die Kanäle werden unter der mit Spannung anliegenden Hülle 4 ohne weitere Befestigung am Schaft des Endoskops 2 fixiert. Die Kanäle liegen im gezeigten Beispiel auf dem Endoskopschaft auf. Alternativ ist denkbar, das die Kanäle 6a-6c und 7 in eine dafür im Endoskop 2 vorgesehene Vertiefung eingelassen sind, so dass die Hülle 4 das Endoskop 2 im Querschnitt kreisrund umgibt.

## Patentansprüche

1. Endoskop (2) mit Hygieneschutz (1), bestehend
- aus einer Hülle (4),
- die am distalen Ende (3) geschlossen sowie zumindest stirnseitig für optische Informationen durchlässig ist, und
- die in Achsrichtung des Endoskops (2) kondomartig aufrollbar ist, und
- aus einem oder mehreren parallel zum Endoskop (2) verlaufenden Arbeitskanälen (6a bis 6b), die am distalen Ende (3) der Hülle (4) offen enden, wobei
- die Arbeitskanäle (6a bis 6b) nur mit dem distalen Ende (3) der Hülle (1) verbunden sind, und
- die Arbeitskanäle (6a bis 6b) zwischen der Außenseite des Endoskops (2) und der Innenseite der Hülle (4) positioniert sind,
**dadurch gekennzeichnet, dass**
- neben den Arbeitskanälen (6a bis 6b) ein oder mehrere Vakuumkanäle (7) mit einer oder mehreren Öffnungen, die auf der dem Endoskop (2) zugewandten Innenseite der Hülle (4) enden, vorgesehen sind,

2. Endoskop nach Anspruch 1, **dadurch gekennzeichnet, dass** es sich um medizinsche Endoskope handelt, die im Querschnitt einen variablen Durchmesser aufweisen.

3. Endoskop nach einem der Ansprüche 1 und 2, **dadurch gekennzeichnet, dass** die Hülle (4) aus einem luft- und wasserdichten sowie für krankheitsbildende Keime undurchlässigem, Material besteht und die Verbindung der Arbeitskanäle (6a bis 6b) mit dem distalen Ende (3) der Hülle (4) luft- und wasserdicht sowie für krankheitsbildende Keime undurchlässig ist.

4. Endoskop nach einem der vorhergehenden Ansprüche, **dadurch gekennzeichnet, dass** die Hülle (4) aus einem elastischen gummiartigen Material besteht, wie es zu medizinischen Zwecken üblicherweise verwendet wird.

5. Endoskop nach einem der vorhergehenden Ansprüche, **dadurch gekennzeichnet, dass** die Hülle (4) flexibel und elastisch sowie in Achsrichtung des Endoskops (2) balgartig zusammenfaltbar ist.

6. Endoskop nach einem der vorhergehenden Ansprüche, **dadurch gekennzeichnet, dass** die Hülle (4) zumindest abschnittsweise einen Innendurchmesser aufweist, der geringfügig größer ist, als der Aussendurchmesser des Endoskops (2).

7. Endoskop nach einem der vorhergehenden Ansprüche, **dadurch gekennzeichnet, dass** die Hülle (4) zumindest die in die Körperöffnungen des Patienten eingeführten Teile des Endoskops (2) umschließt.

8. Endoskop nach einem der vorhergehenden Ansprüche, **dadurch gekennzeichnet, dass** am distalen Ende (3) der Hülle (4) stirnseitig eine transparente Scheibe oder eine Linse vorgesehen ist.

9. Endoskop nach Anspruch 8, **dadurch gekennzeichnet, dass** die Scheibe oder die Linse; ganz oder teilweise, die Stirnseite des distalen Endabschnitts (3) bildet.

10. Endoskop nach einem der vorhergehenden Ansprüche, **dadurch gekennzeichnet, dass** der distale Endabschnitt (3) der Hülle (4) als optisch transparente Kappe (3) ausgebildet ist.

11. Endoskop nach Anspruch 10, **dadurch gekennzeichnet, dass** die Wandstärke des kappenartigen distalen Endabschnitts (3) größer ist, als die Wandstärke der Hülle (4) im nicht-distalen Bereich.

12. Endoskop nach einem der vorhergehenden Ansprüche, **dadurch gekennzeichnet, dass** die am proximalen Ende offene Hülle (4) am Schaft des Endoskops (2) luftdicht fixierbar ist.

13. Endoskop nach Anspruch 12, **dadurch gekennzeichnet, dass** die Hülle (4) im Bereich des proximalen Endes konisch aufgeweitet ist und der in diesem Bereich faltenfrei umgeschlagene Teil der Hülle mittels eines chemisch inerten und nicht toxischen Adhäsionsklebers fixierbar ist.

14. Endoskop nach einem der vorhergehenden Ansprüche, **dadurch gekennzeichnet, dass** parallel zum Endoskop auf der Innenseite der Hülle (4) über deren gesamte Länge oder einen Teil ihrer Länge ein an seinem distalen Ende mit der Hülle verbundener Reißfaden verläuft.

15. Endoskop nach einem der vorhergehenden Ansprüche, **dadurch gekennzeichnet, dass** mindestens einer der Arbeitskanäle (6a bis 6c) zur Einleitung von flüssigkeiten und/oder Luft vorgesehen ist.

16. Endoskop einem der vorhergehenden Ansprüche, **dadurch gekennzeichnet, dass** mindestens einer der Arbeitskanäle (6a bis 6c) bei entsprechender Wandstärke als Saugleitung zur Entnahme von Körperflüssigkeiten vorgesehen ist.

17. Endoskop nach einem der vorhergehenden Ansprüche, **dadurch gekennzeichnet, dass** mindestens einer der Arbeitskanäle (6a bis 6c) zur Führung von Geräten, die der Entnahme von Biopsien dienen, vorgesehen ist.

18. Endoskop nach einem der vorhergehenden Ansprüche, **dadurch gekennzeichnet, dass** sich der oder die Vakuumkanäle (7) über die gesamte Länge der Hülle (4) oder einen Teil der Hülle (4) erstrecken.

19. Endoskop nach einem der vorhergehenden Ansprüche, **dadurch gekennzeichnet, dass** an dem oder den Vakuumkanälen (7) während der Nutzung des Endoskops (2) ein Unterdruck anlegbar ist.

20. Endoskop nach einem der vorhergehenden Ansprüche, **dadurch gekennzeichnet, dass** die Aussenseite des Endoskops (2) in axialer Richtung Vertiefungen aufweist, die in Form und Tiefe dem Durchmesser und Profil der Arbeits- (6a bis 6c) und Vakuumkanäle entsprechen.

21. Endoskop nach Anspruch 20, **dadurch gekennzeichnet, dass** die Breite der in axialer Richtung auf der Aussenseite des Endoskops (2) verlaufenden Vertiefungen geringfügig geringer bemessen ist, als die Breite der Vertiefung in ihrem Mittelpunkt.

22. Endoskop nach einem der vorhergehenden Ansprüche, **dadurch gekennzeichnet, dass**, die Arbeits- (6a bis 6b) und Vakuumkanäle mit dem distalen Ende der Hülle (4) fest und/oder lösbar verbunden sind.

23. Endoskop nach Anspruch 22, **dadurch gekennzeichnet, dass** die lösbaren Arbeits- (6a bis 6b) und Vakuumkanäle am proximalen und/oder distalen Ende der Hülle luftdichte und wasserdichte Verbindungselemente aufweisen.

24. Endoskop nach einem der Ansprüche 22 und 23, **dadurch gekennzeichnet, dass** die Verbindungselemente der Arbeits- (6a bis 6b) und Vakuumkanäle am proximalen Ende mit den in der Endoskopie gängigen Ventilanschlüssen kompatibel sind.

25. Endoskop nach einem der vorhergehenden Ansprüche, **dadurch gekennzeichnet, dass** die Arbeits- (6a bis 6b) und Vakuumkanäle am proximalen Ende mit einer Steuereinheit zur Bedienung der Endoskopiekanäle verbunden sind.

26. Endoskop nach Anspruch 25, **dadurch gekennzeichnet, dass** die Steuereinheit zur Bedienung der Endoskopiekanäle vorzugsweise einmaligen Nutzung vorgesehen und aus Plastik gestaltet ist.

27. Verfahren zur Anbringung eines Hygieneschutzes an einem medizinschen Endoskop nach einem der Ansprüche 1 bis 19 und 22 bis 26, **dadurch gekennzeichnet, dass**
- das Fenster am distalen Ende der Hülle (4) innenseitig mit einem Stoff beaufschlagt wird, der den optischen Kontakt zwischen Fenster und dem optischen Kanal des Endoskops (2) herstellt,
- das distale Ende des Endoskops (2) in die am proximalen Ende offene und am distalen Ende geschlossene Hülle (4) eingeführt wird,
- die kondomartig aufgerollte oder balgartig zusammengefaltete Hülle (4) unter Einschluß des Endoskops (2) und der Arbeitskanäle (6a bis 6c) abgerollt oder entfaltet wird, und
- am Vakuumschlauch ein Unterdruck angelegt wird.

28. Verfahren zur Anbringung eines Hygieneschutzes an einem medizinschen Endoskop nach einem der Ansprüche 20 und 21 **dadurch gekennzeichnet, dass**
- das Fenster am distalen Ende der Hülle (4) innenseitig mit einem Stoff beaufschlagt wird, der den optischen Kontakt zwischen Fenster und dem optischen Kanal des Endoskops (2) herstellt,
- das distale Ende des Endoskops (2) in die am proximalen Ende offene und am distalen Ende geschlossene Hülle (4) eingeführt wird,
- die am distalen Ende der Hülle befestigten Arbeitskanäle (6a-6c) in den auf der Aussenseite des Endoskops (2) vorgesehenen Vertiefungen positioniert werden,
- die kondomartig aufgerollte oder balgartig zusammengefaltete Hülle (4) unter Einschluß des Endoskops (2) und der Arbeitskanäle (6a bis 6c) abgerollt oder entfaltet wird,
- am Vakuumschlauch ein Unterdruck angelegt wird.

## Claims

1. Endoscope (2) with hygiene protection (1), which comprises
- a cover (4),
- which is closed at the distal end (3) and is transmissible for optical information at least at its front face, and
- which can be unfurled in the manner of a condom in the axial direction of the endoscope (2), and
- consists of one or more working channels (6a to 6b) which extend parallel to the endoscope (2) and terminate in an open manner at the distal end (3) of the cover (4),
- the working channels (6a to 6b) are only connected to the distal end (3) of the cover (1), and
- the working channels (6a to 6b) are positioned between the outer side of the endoscope (2) and the inside of the cover (4).
**characterised in that**
- in addition to the working channels (6a to 6b), one or more vacuum channels (7) with one or more openings, which terminate at the inside of the cover (4), which faces the endoscope (2), are provided.

2. Endoscope according to claim 1, **characterised in that** it is a medical endoscope which has a variable diameter in the cross-section

3. Endoscope according to one of claims 1 and 2, **characterised in that** the cover (4) consists of a material that is airtight and watertight and is impermeable to pathological microorganisms, and the connection of the working channels (6a to 6b) to the distal end (3) of the cover (4) is airtight and watertight and is impermeable to pathological microorganisms.

4. Endoscope according to one of the preceding claims, **characterised in that** the cover (4) consists of an elastic rubber-like material as is conventionally used for medical purposes.

5. Endoscope according to one of the preceding claims, **characterised in that** the cover (4) is flexible and elastic and can be folded up in the manner of a bellows in the axial direction of the endoscope (2).

6. Endoscope according to one of the preceding claims, **characterised in that** the cover (4), at least in some sections, has an internal diameter that is slightly larger than the external diameter of the endoscope (2).

7. Endoscope according to one of the preceding claims, **characterised in that** the cover (4) encloses at least those parts of the endoscope (2) that are introduced into the body orifices of the patient.

8. Endoscope according to one of the preceding claims, **characterised in that** a transparent pane or a lens is provided on the distal end (3) of the cover (4) at its front face.

9. Endoscope according to claim 8, **characterised in that** the pane or the lens entirely or partly forms the front face of the distal end section (3).

10. Endoscope according to one of the preceding claims, **characterised in that** the distal end section (3) of the cover (4) is designed as an optically transparent cap (3)..

11. Endoscope according to claim 10, **characterised in that** the wall thickness of the cap-like distal end section (3) is greater than the wall thickness of the cover (4) in the non-distal region.

12. Endoscope according to one of the preceding claims, **characterised in that** the cover (4), which is open at the proximal end, can be fixed so as to be airtight on the shaft of the endoscope (2).

13. Endoscope according to claim 12, **characterised in that** the cover (4) is conically enlarged in the region of the proximal end and the part of the cover that is folded back so as to be wrinkle-free in this region can be fixed by means of a chemically inert and non-toxic adhesive.

14. Endoscope according to one of the preceding claims, **characterised in that** a tear thread, which is connected to the cover (4) at the distal end of the endoscope, runs parallel to the endoscope on the inside of the cover, over the entire length or a part of the length thereof.

15. Endoscope according to one of the preceding claims, **characterised in that** at least one of the working channels (6a to 6c) is provided for introducing liquids and/or air.

16. Endoscope according to one of the preceding claims, **characterised in that** at least one of the working channels (6a to 6c), with an appropriate wall thickness, is provided as a suction line for removing body fluids.

17. Endoscope according to one of the preceding claims, **characterised in that** at least one of the working channels (6a to 6c) is provided for guiding equipment that serve for the removal of biopsies.

18. Endoscope according to one of the preceding claims, **characterised in that** the vacuum channel or vacuum channels (7) extend over the entire length of the cover (4) or a part of the cover (4).

19. Endoscope according to one of the preceding claims, **characterised in that** a subatmospheric pressure is applied to the vacuum channel or vacuum channels (7) during the use of the endoscope (2)..

20. Endoscope according to one of the preceding claims, **characterised in that** the outside of the endoscope (2) has depressions in the axial direction, which correspond in shape and depth to the diameter and profile of the working (6a to 6c) and vacuum channels.

21. Endoscope according to claim 20, **characterised in that** the width of the depressions running in the axial direction on the outside of the endoscope (2) is slightly smaller in dimension that the width of the depression in its centre point.

22. Endoscope according to one of the preceding claims, **characterised in that** the working (6a to 6b) and vacuum channels are firmly and/or detachably connected to the distal end of the cover (4).

23. Endoscope according to claim 22, **characterised in that** the detachable working (6a to 6b) and vacuum channels have airtight and watertight connecting elements at the proximal and/or distal end of the cover..

24. Endoscope according to one of claims 22 and 23, **characterised in that** the connecting elements of the working (6a to 6b) and vacuum channels are compatible at the proximal end with the valve connections that are conventional in endoscopy..

25. Endoscope according to one of the preceding claims, **characterised in that** the working (6a to 6b) and vacuum channels are connected to a control unit for operating the endoscopy channels.

26. Endoscope according to claim 25, **characterised in that** the control unit for operating the endoscopy channels is preferably provided for one-off use and is made of plastic.

27. Method for attaching a hygiene protection to a medical endoscope according to one of claims 1 to 19 and 22 to 26, **characterised in that**
- the window at the distal end of the cover (4) is coated on the inside with a material that produces optical contact between the window and the optical channel of the endoscope (2)
- the distal end of the endoscope (2) is introduced into the cover (4), which is open at the proximal end and closed at the distal end
- the cover (4), which is rolled up in the manner of a condom or folded up in the manner of bellows, is unfurled or unfolded with inclusion of the endoscope (2) and the working channels (6a to 6c)
- a subatmospheric pressure is applied to the vacuum tube

28. Method for attaching a hygiene protection to a medical endoscope according to one of claims 20 and 21, **characterised in that**
- the window at the distal end of the cover (4) is coated on the inside with a material that produces optical contact between the window and the optical channel of the endoscope (2)
- the distal end of the endoscope (2) is introduced into the cover (4), which is open at the proximal end and closed at the distal end
- the working channels (6a-6c), which are fixed at the distal end, are positioned in the depressions provided at the outside of the endoscope (2)
- the cover (4), which is rolled up in the manner of a condom or folded up in the manner of bellows, is unfurled or unfolded with inclusion of the endoscope (2) and the working channels (6a to 6c)
- a subatmospheric pressure is applied to the vacuum tube

## Revendications

1. Endoscope (2) avec protection hygiénique (1), constitué
• d'une enveloppe (4),
• qui est fermée à l'extrémité distale (3) et est perméable, au moins sur sa face frontale, aux informations optiques, et
• qui peut être enroulée dans la direction de l'axe de l'endoscope (2) à la manière d'un condom interne, et
• d'un ou deux canaux de travail (6a à 6b) courant parallèlement à l'endoscope (2)) (2), qui se terminent de manière ouverte à l'extrémité distale (3) de l'enveloppe (4), sachant que
• les canaux de travail (6a à 6b) ne sont reliés qu'avec l'extrémité distale (3) de l'enveloppe (1), et
• que les canaux de travail (6a à 6b) sont positionnés entre la face extérieure de l'endoscope (2) et la face intérieure de l'enveloppe (4),
**caractérisé par le fait**
• **qu'**un ou plusieurs canaux de vide avec une ou plusieurs ouvertures se terminant sur là face intérieure de l'enveloppe tournée vers l'endoscope (2) sont prévus.

2. Endoscope selon la revendication 1, **caractérisé par le fait qu'**il s'agit d'un endoscope médical présentant un diamètre variable dans sa section.

3. Endoscope selon une des revendications 1 et 2, **caractérisé par le fait que** l'enveloppe (4) consiste en un matériau étanche à l'air et à l'eau et imperméable aux souches infectieuses et que la liaison des canaux de travail (6a à 6b) avec l'extrémité distale (3) de l'enveloppe (4) est étanche à l'air et à l'eau et imperméables aux souches infectieuses.

4. Endoscope selon une des revendications précédentes, **caractérisé par le fait que** l'enveloppe (4) consiste en un matériau élastique semblable à du caoutchouc, comme il en est utilisé habituellement en médecine.

5. Endoscope selon une des revendications précédentes, **caractérisé par le fait que** l'enveloppe (4) est souple et élastique est peut être repliée sur elle-même à la manière d'un soufflet dans la direction de l'axe de l'endoscope.

6. Endoscope selon une des revendications précédentes, **caractérisé par le fait que** l'enveloppe (4) présente, au moins par tronçon, un diamètre intérieur qui est légèrement plus grand que le diamètre extérieur de l'endoscope (2).

7. Endoscope selon une des revendications précédentes, **caractérisé par le fait que** l'enveloppe (4) comprend au moins les parties de l'endoscope introduites dans les ouvertures corporelles du patient.

8. Endoscope selon une des revendications précédentes, **caractérisé par le fait qu'**un disque transparent ou une lentille est prévue sur la face frontale de l'extrémité distale (3) de l'enveloppe (4).

9. Endoscope selon la revendication 8, **caractérisé par le fait que** le disque ou la lentille forme complètement ou partiellement la face frontale du tronçon distal final (3).

10. Endoscope une des revendications précédentes, **caractérisé par le fait que** le tronçon distal final (3) de l'enveloppe (4) a la forme d'un capuchon (3) optiquement transparent.

11. Endoscope selon la revendication 10, **caractérisé par le fait que** l'épaisseur de paroi du tronçon distal final ayant la forme d'un capuchon est plus grand que l'épaisseur de paroi de l'enveloppe (4) dans la zone non-distale.

12. Endoscope selon une des revendications précédentes, **caractérisé par le fait que** l'enveloppe ouverte à l'extrémité proximale peut être fixée de façon étanche à l'air sur la tige de l'endoscope (2).

13. Endoscope selon la revendication 12, **caractérisé par le fait que** l'enveloppe (4) est élargie de façon conique dans la zone de l'extrémité proximale et que la partie rabattue sans pli de l'enveloppe dans cette zone peut être fixée au moyen d'une colle adhésive inerte au plan chimique et non toxique.

14. Endoscope selon une des revendications précédentes, **caractérisé par le fait qu'**un fil de déchirement relié à l'enveloppe sur la face intérieure de l'enveloppe court parallèlement à l'endoscope sur la face intérieure de l'enveloppe (4) sur toute la longueur de cette dernière ou sur une partie de sa longueur.

15. Endoscope selon une des revendications précédentes, **caractérisé par le fait qu'**au moins un des canaux de travail (6a à 6c) est prévu pour l'introduction de liquides et / ou d'air.

16. Endoscope selon une des revendications précédentes, **caractérisé par le fait qu'**au moins un des canaux de travail (6a à 6c) est prévu, avec l'épaisseur de paroi correspondante, en tant en tant que conduite d'aspiration pour le prélèvement de liquides corporels.

17. Endoscope selon une des revendications précédentes, **caractérisé par le fait qu'**au moins un des canaux de travail (6a à 6c) est prévu pour l'introduction d'appareils servant au prélèvement de biopsies.

18. Endoscope selon une des revendications précédentes, **caractérisé par le fait que** le ou les canaux de vide (7) s'étendent sur toute ou partie de la longueur de l'enveloppe (4) ou d'une partie de l'enveloppe (4).

19. Endoscope selon une des revendications précédentes, **caractérisé par le fait qu'**une sous-pression peut être créée sur le ou les canaux de vide (7) pendant l'utilisation de l'endoscope,

20. Endoscope selon une des revendications précédentes, **caractérisé par le fait que** la face extérieure de l'endoscope (2) présente en direction de l'axe des renfoncements correspondant dans leur forme et dans leur profondeur au diamètre et au profil des canaux de travail (6a à 6c) et de vide.

21. Endoscope selon la revendication 20, **caractérisé par le fait que** la largeur des renfoncements courant dans la direction de l'axe sur la face extérieure de l'endoscope (2) est légèrement plus faible que la largeur du renfoncement dans son point médian.

22. Endoscope selon une des revendications précédentes, **caractérisé par le fait que** les canaux de travail (6a à 6b) et les canaux de vide sont reliés de façon fixe ou pouvant être détachés avec l'extrémité distale de l'enveloppe (4).

23. Endoscope selon la revendication 22, **caractérisé par le fait que** les canaux de travail (6a à 6b) et les canaux de vide présentent des éléments de liaison étanches à l'air et étanches à l'eau sur l'extrémité proximale et / ou distale.

24. Endoscope selon une des revendications 22 et 23, **caractérisé par le fait que** les éléments de liaison des canaux de travail (6 a à 6b) et les canaux de vide sont compatibles à l'extrémité proximale avec les raccordements de valve usuels en endoscopie

25. Endoscope selon une des revendications précédentes, **caractérisé par le fait que** les canaux de travail (6a à 6b) et de vide sur l'extrémité proximale sont reliés avec une unité de commande pour l'utilisation de canaux d'endoscopie.

26. Procédé selon la revendication 25, **caractérisé par le fait que** l'unité de commande pour l'utilisation des canaux d'endoscopie est prévue pour un usage unique et est réalisée en plastique.

27. Procédé pour la pose d'une protection hygiénique sur un endoscope médical selon une des revendications 1 à 19 et 22 à 26, **caractérisé par le fait que**
• la fenêtre sur l'extrémité distale de l'enveloppe (4) est injectée à l'aide d'une matière créant le contact optique entre la fenêtre et le canal optique de l'endoscope (2),
• l'extrémité distale de l'endoscope est introduite dans l'enveloppe ouverte à l'extrémité proximale et fermée à l'extrémité distale,
• l'enveloppe (4) enroulée ayant la forme d'un condom ou repliée sur elle-même ayant la forme d'un soufflet est déroulée ou dépliée en incluant l'endoscope (2) et les canaux de travail (6a à 6c),
• et une sous-pression étant créée sur le flexible de vide.

28. Procédé pour la pose d'une protection hygiénique sur un endoscope médical selon une des revendications 20 et 21, **caractérisé par le fait que**
• la fenêtre est injectée intérieurement à l'extrémité distale de l'enveloppe avec une matière créant le contact optique entre la fenêtre et le canal optique de l'endoscope (2),
• l'extrémité distale de l'endoscope (2) est introduite dans l'enveloppe fermée ouverte à l'extrémité proximale et fermée à l'extrémité distale,
• les canaux de travail (6a-6c) fixés à l'extrémité distale de l'enveloppe sont positionnés dans les renfoncements prévus sur la face extérieure de l'endoscope (2),
• l'enveloppe (4) enroulée ayant la forme d'un condom ou repliée sur elle-même ayant la forme d'un soufflet est déroulée ou dépliée en incluant l'endoscope (2),
• une sous-pression étant créée sur le flexible de vide.
